# FASCICULE DE BREVET EUROPEEN

(11) **EP 0 817 626 B1**
(45) Date de publication et mention de la délivrance du brevet: **28.10.1998**
(21) Numéro de dépôt: 96909200.6
(22) Date de dépôt: 26.03.1996
(51) Int. Cl.: A61K 31/335

(54) **UTILISATION DU FUMAGILLOL ET SES DERIVES POUR PREPARER LES MEDICAMENTS DESTINES A COMBATTRE LES INFECTIONS INTESTINALES**
VERWENDUNG VON FUMAGILLOL UND SEINEN DERIVATEN ZUR HERSTELLUNG VON ARZNEIMITTELN GEGEN DARMINFEKTIONEN
USE OF FUMAGILLOL AND DERIVATIVES THEREOF FOR PREPARING MEDICAMENTS AGAINST INTESTINAL INFECTIONS

(30) Priorité: 27.03.1995 FR 9503549
(43) Date de publication de la demande: 14.01.1998
(73) Titulaire: SANOFI, 75008 Paris (FR); ASSISTANCE PUBLIQUE, HOPITAUX DE PARIS, 75004 Paris (FR)
(72) Inventeur: MOLINA, Jean-Michel, 75015 Paris (FR); DEROUIN, Francis, 78100 Saint-Germain-en-Laye (FR)
(74) Mandataire: Gillard, Marie-Louise
(86) Numéro de dépôt international: FR9600448
(87) Numéro de publication internationale: WO9630010

(56) Documents cités:
- WO-A-94/19946
- CORNEA, MAY 1993, 12 (3) P261-5, UNITED STATES, XP000603990 ROSBERGER DF ET AL: "Successful treatment of microsporidial keratoconjunctivitis with topical fumagillin in a patient with AIDS."
- AM J OPHTHALMOL, MAR 15 1993, 115 (3) P293-8, UNITED STATES, XP000604167 DIESENHOUSE MC ET AL: "Treatment of microsporidial keratoconjunctivitis with topical fumagillin"
- INFECT. DIS. CLIN. NORTH AM., 1994, 8/2 (483-498), USA, XP000604170 MANNHEIMER S.B. ET AL: "Protozoal infections in patients with AIDS: Cryptosporidiosis, isosporiasis, cyclosporiasis, and microsporidiosis"
- PATENT ABSTRACTS OF JAPAN vol. 011, no. 167 (C-425), 28 Mai 1987 & JP,A,62 000476 (FUJISAWA PHARMACEUT CO LTD), 6 Janvier 1987,
- CANCER RESEARCH, vol. 55, no. 4, 15 Février 1995, pages 836-839, XP000605152 TANAKA, T. ET AL: "Prevention of hepatic metastasis of human colon cancer by angiogenesis inhibitor TNP-470"
- REV. MED. MICROBIOL., 1992, 3/1 (35-42), UNITED KINGDOM, XP000604169 CANNING E.U. ET AL: "Human infections with microsporidia"

## Description

La présente invention concerne une nouvelle utilisation du fumagillol et de ses dérivés, notamment de la fumagilline.

La fumagilline est un antibiotique décrit pour la première fois en 1951 (The Merck Index 11th Edition, n. 4199), utilisé surtout pour prévenir ou contrôler les maladies parasitaires dans les élevages de poissons et d'abeilles, qui a été également utilisé chez l'homme pour le traitement local, dans un collyre, d'une kérato-conjonctivite due à *Encephalitozoon hellem* (J. Ophtal., 1993, 115, 293). Elle a été cependant trouvée inactive comme cancérolytique (Antibiotic Annual, 1958-1959, 541-546).

Il a été maintenant trouvé que le fumagillol et certains esters formés avec ce composé, formulés dans un médicament, notamment un médicament par voie orale, sont capables de resoudre des états infectieux très graves de l'intestin dûs à des microsporidies ou à des cryptosporidies.

Il a été également trouvé, de façon surprenante, que le fumagillol et les esters formés par le fumagillol et des acides (C₁-C₁₂)alkylcarboxyliques ou (C₁-C₁₂)alkyldicarboxyliques, saturés ou insaturés, sont capables d'induire une éradication de l'*Enterocytozoon bieneusi* chez des patients atteints par le virus HIV. Cette découverte est surprenante et décisive parce que pour ce type d'infection, qui constitue 95% des infections intestinales à microsporidies conduisant à la cachexie et à la mort les malades atteints du SIDA, aucun remède n'est connu à l'heure actuelle.

Ainsi, la présente invention concerne, selon un de ses aspects, l'utilisation du fumagillol ou des esters formés par le fumagillol et des acides (C₁-C₁₂)alkylcarboxyliques ou (C₁-C₁₂)alkyldicarboxyliques, saturés ou insaturés, et de leurs sels pharmaceutiquement acceptables pour la préparation de médicaments destinés à combattre les infections intestinales dues à des microsporidies et/ou à des cryptosporidies.

Plus particulièrement et de façon avantageuse, la présente invention concerne, selon son aspect préféré, l'utilisation du fumagillol ou des esters formés par le fumagillol et des acides (C₁-C₁₂)alkylcarboxyliques ou (C₁-C₁₂)alkyldicarboxyliques, saturés ou insaturés et de leurs sels pharmaceutiquement acceptables pour la préparation de médicaments destinés à combattre les infections intestinales dont le responsable principal est le parasite *Enterocytozoon bieneusi*.

Par "acides (C₁-C₁₂)alkylcarboxyliques ou (C₁-C₁₂)alkyldicarboxyliques, saturés ou insaturés" on entend des acides carboxyliques ou dicarboxyliques d'alkyles linéaires ou ramifiés, lesdits alkyles pouvant porter une ou plusieurs doubles liaisons.

De tels acides sont par exemple les acides acétique, propionique, butyrique, valérique, pivalique, malonique, succinique, acrylique, crotonique, isocrotonique, oléique, maléique, fumarique, 2,4,6,8-decatétraènedioïque.

L'ester du fumagillol et de l'acide 2,4,6,8-décatétraènedioïque, la fumagilline, est un composé particulièrement avantageux.

Les esters de la présente invention sont aisément préparés par réaction entre le fumagillol et l'acide approprié dans les conditions normales d'estérification décrites en littérature.

Le fumagillol, tel quel ou estérifié avec un acide (C₁-C₁₂)alkylcarboxylique ou (C₁-C₁₂)alkyldicarboxylique, peut être administré sous forme d'acide libre ou bien sous forme d'un de ses sels avec une base pharmaceutiquement acceptable.

Pour leur administration à des patients atteints d'une infection due à des microsporidies ou à des cryptosporidies, le fumagillol ou les esters formés avec celui-ci sont mélangés à des excipients pharmaceutiques couramment utilisés pour la préparation de formulations pharmaceutiques, de préférence pour l'administration orale.

Avantageusement, les composés de la présente invention sont formulés, en tant que principes actifs, en unités de dosage, par exemple comprimés ou gélules, contenant de 1 à 200 mg de principe actif avantageusement de 2 à 100 mg, mieux de 5 à 50 mg ou, de préférence, de 7,5 à 30 mg par unité de dosage.

Les compositions pharmaceutiques pour l'administration orale constituent un objet ultérieur de la présente invention.

Dans les compositions pharmaceutiques de la présente invention pour l'administration orale, le principe actif peut être administré sous les formes unitaires d'administration sus-dites, en mélange avec des supports pharmaceutiques classiques, pour le traitement des affections sus-mentionnées. Les formes unitaires d'administration appropriées comprennent les formes par voie orale telles que les comprimés éventuellement sécables, les gélules, les poudres, les granulés et les solutions ou suspensions orales.

Lorsqu'on prépare une composition solide sous forme de comprimés, une des formes préférées, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif. Ces comprimés à action retard ou à libération contrôlée constituent une autre forme très avantageuse.

On obtient une préparation en gélules, une autre forme particulièrement avantageuse, en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs de goût.

Le principe actif peut être formule également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

Des compositions pharmaceutiques par voie orale contenant de 1 à 200 mg, de préférence de 2 à 100 mg, de 5 à 50 mg ou de 7,5 à 30 mg de fumagillol, d'un ester formé par le fumagillol et un des acides mentionnés ci-dessus, notamment la fumagilline ou d'un de leurs sels pharmaceutiquement acceptables en tant que principe actif sous une forme choisie parmi les comprimés, les comprimés retard, les comprimés à libération contrôlée et les gélules constituent un objet ultérieur de la présente invention.

Les composés de la présente invention peuvent être administrés avec d'autres médicaments généralement utilisés pendant le développement du SIDA et peuvent également être formulés en association avec d'autres antiparasitaires ou antibiotiques ou avec des médicaments à action anti-HIV.

L'activité thérapeutique des composés de la présente invention a été démontrée en administrant des doses différentes de la composition pharmaceutique de l'Exemple 1 à quatre patients de sexe masculin, homosexuels, présentant un déficit immunitaire important avec un taux de CD4 moyen à 66 (11-158). Trois patients avaient un SIDA avéré et un patient un ARC (de l'anglais *Aids Related Complex*). L'age moyen était de 40 ans.

Tous ces patients avaient en pré-inclusion des examens de selles consécutifs positifs aux microsporidies. Deux patients présentaient de façon concomitante une infection intestinale à cryptosporidies. La recherche de microsporidies dans les urines est restée négative chez tous les patients, éléments en faveur du diagnostic d'infection à *Enterocytozoon bieneusi*.

Tous les patients ont eu une fibroscopie duodénale avec biopsies. Chez trois patients des microsporidies ont été identifiées sur ces biopsies en histologie et en parasitologie directe ainsi que par microscopie électronique. Dans le quatrième cas, seule la microscopie électronique a revelé la présence d'*Enterocytozoon bieneusi*.

Les quatre patients ont reçu 20 mg de fumagilline trois fois par jour, soit 60 mg/j pendant 21 jours.

Une éradication du parasite des selles a été observée chez tous les patients aux examens de contrôle après 15, 17 et 21 jours de traitement. Un mois après l'arrêt du traitement, on ne retrouvait toujours pas de microsporidies dans les selles des patients.

Parmi les deux patients qui présentaient des cryptosporidies dans leurs selles en pré-inclusion, un seul a négativé ses selles de façon transitoire avec une réapparition des cryptosporidies un mois après l'arrêt du traitement.

Tous les patients ont eu une fibroscopie duodénale de contrôle pour apprécier la disparition des parasites au niveau tissulaire. De plus, on a observé une négativation sur le plan histologique avec persistance de très rares microsporidies en parasitologie directe dans deux cas (probablement des cadavres de microsporidies). L'étude en microscopie électronique a confirmé chez les quatre patients la disparition totale de l'*Enterocytozoon bieneusi*.

Un autre groupe de patients a été recruté pour un essai de tolérance/toxicologie. Des doses de 10, 20, 40 et 60 mg de fumagilline ont été administrées à vingt quatre patients (six par palier de dose).

Le traitement a été bien toléré. Notamment, aucune toxicité hépatique, cardiaque (ECG) ou rénale (créatininémie) n' a été observée. On a noté même une discrète diminution du taux sérique des phosphatases alcalines qui pourrait correspondre à un effet bénéfique du traitement sur la cholangite à microsporidies.

L'effet secondaire génant observé a été du type hématologique, à savoir des thrombopénies de degré variable mais jamais très sévères (sauf dans un cas qui a causé l'interruption de l'administration dans le groupe des quatre patients ci-dessus) et d'évolution spontanément régressive à l'arrêt du traitement en 10 à 14 jours. Cet effet secondaire, qui n'est pas immunologique mais uniquement dû à une toxicité directe sur les plaquettes peut être corrigé par un choix convenable du protocole de traitement.

En conclusion, il a été observé pour la première fois dans cette infection opportuniste une éradication du parasite des selles, éradication persistant au moins un mois après l'arrêt du traitement, et qui semble s'accompagner d'une éradication du parasite des biopsies duodénales. Ce résultat n'a jamais été obtenu avec d'autres antiparasitaires.

Le bénéfice clinique est difficile à évaluer chez ces patients compte tenu des multiples infections associées et de la poursuite des traitements symptomatiques. Un bénéfice clinique très net a été néanmoins obtenu chez les patients traités qui ont tous récupéré plusieurs kilogrammes et qui ont vu leur diarrhée s'arrêter à la fin du traitement.

Par conséquent, avec la fumagilline on a obtenu des résultats parasitologiques spectaculaires dans les infections à *Enterocytozoon bieneusi* au cours du SIDA, dans certains cas, un excellent résultat clinique.

### EXAMPLE 1

### Composition pharmaceutique en gélules contenant chacune 20 mg de fumagilline acide.

Une quantité de 14 g de fumagilline acide purifiée et préalablement tamisée est progressivement diluée avec la quantité nécessaire de silice colloïdale (AEROSIL®) jusqu'à un volume de 210 ml. La poudre ainsi obtenue est bien mélangée et la poudre homogène ainsi préparée est répartie dans des gélules de gélatine dure opaque de taille n° 3. On obtient ainsi 700 gélules contenant chacune 20 mg de fumagilline acide.

## Revendications

1. Utilisation du fumagillol ou d'un ester formé par le fumagillol et un acide (C₁-C₁₂)alkylcarboxylique ou (C₁-C₁₂)alkyldicarboxylique, saturé ou insaturé, et de leurs sels pharmaceutiquement acceptables pour la préparation de médicaments destinés à combattre les infections intestinales dues à des microsporidies et/ou à des cryptosporidies.

2. Utilisation selon la revendication 1 où ledit ester est la fumagilline.

3. Utilisation du fumagillol ou d'un ester formé par le fumagillol et un acide (C₁-C₁₂)alkylcarboxylique ou (C₁-C₁₂)alkyldicarboxylique, saturé ou insaturé, et de leurs sels pharmaceutiquement acceptables selon la revendication 1, pour la préparation de médicaments destinés à combattre les infections intestinales dont le responsable principal est le parasite *Enterocytozoon bieneusi*.

4. Utilisation selon la revendication 3 où ledit ester est la fumagilline.

5. Utilisation selon la revendication 1 ou 2, pour la préparation de médicaments pour l'administration orale.

6. Utilisation selon la revendication 3 ou 4, pour la préparation de médicaments sous forme de comprimés, de gélules ou de comprimés retard ou à libération contrôlée.

7. Composition pharmaceutique pour administration orale contenant un ester formé par le fumagillol et un acide (C₁-C₁₂)alkyldicarboxylique, saturé ou insaturé, ou un de ses sels pharmaceutiquement acceptables en tant que principe actif.

8. Composition pharmaceutique selon la revendication 7, où ledit ester est la fumagilline.

9. Composition pharmaceutique selon la revendication 7 ou 8, contenant de 1 à 200 mg de principe actif.

10. Composition pharmaceutique selon la revendication 9, contenant de 7,5 à 30 mg de principe actif.

## Claims

1. Use of fumagillol or an ester formed by fumagillol and a saturated or unsaturated (C₁-C₁₂)alkylcarboxylic or (C₁-C₁₂)alkyldicarboxylic acid, and their pharmaceutically acceptable salts, for the preparation of drugs for combating intestinal infections due to microsporidia and/or cryptosporidia.

2. Use according to claim 1 wherein said ester is fumagillin.

3. Use of fumagillol or an ester formed by fumagillol and a saturated or unsaturated (C₁-C₁₂)alkylcarboxylic or (C₁-C₁₂)alkyldicarboxylic acid, and their pharmaceutically acceptable salts according to claim 1, for the preparation of drugs for combating intestinal infections for which the parasite *Enterocytozoon bieneusi* is principally responsible.

4. Use according to claim 3 wherein said ester is fumagillin.

5. Use according to claim 1 or 2 for the preparation of drugs for oral administration.

6. Use according to claim 3 or 4 for the preparation of drugs in the form of tablets, gelatin capsules or delayed action or controlled release tablets.

7. Pharmaceutical composition for oral administration, containing an ester formed by fumagillol and a saturated or unsaturated (C₁-C₁₂)alkyldicarboxylic acid, or one of its pharmaceutically acceptable salts, as the active principle.

8. Pharmaceutical composition according to claim 7 wherein said ester is fumagillin.

9. Pharmaceutical composition according to claim 7 or 8 containing from 1 to 200 mg of active principle.

10. Pharmaceutical composition according to claim 9 containing from 7.5 to 30 mg of active principle.

## Patentansprüche

1. Verwendung von Fumagillol oder einem Ester, der aus Fumagillol und einer gesättigten oder ungesättigten (C₁-C₁₂)Alkylcarbon- oder (C₁-C₁₂)Alkyldicarbonsäure gebildet ist, und ihrer pharmazeutisch annehmbarer Salze bei der Herstellung von Medikamenten zur Bekämpfung von Darminfektionen, die durch Microsporidia und/oder Cryptosporidia hervorgerufen werden.

2. Verwendung nach Anspruch 1, wobei der Ester Fumagillin ist.

3. Verwendung von Fumagillol oder einem Ester, der aus Fumagillol und einer gesättigten oder ungesättigten (C₁-C₁₂)Alkylcarbon- oder (C₁-C₁₂)Alkyldicarbonsäure gebildet ist, und ihrer pharmazeutisch annehmbarer Salze nach Anspruch 1 bei der Herstellung von Medikamenten zur Bekämpfung von Darminfektionen, für die hauptsächlich der Parasit Enterocytozoon bieneusi verantwortlich ist.

4. Verwendung nach Anspruch 3, wobei der Ester Fumagillin ist.

5. Verwendung nach Anspruch 1 oder 2 bei der Herstellung von Medikamenten zur oralen Verabreichung.

6. Verwendung nach Anspruch 3 oder 4 bei der Herstellung von Medikamenten in Form von Tabletten, Kapseln oder Retard-Tabletten oder Tabletten mit gesteuerter Freisetzung.

7. Pharmazeutische Zusammensetzung zur oralen Verabreichung, welche einen aus Fumagillol und einer gesättigten oder ungesättigten (C₁-C₁₂)Alkyldicarbonsäure gebildeten Ester oder eines seiner pharmazeutisch annehmbaren Salze als aktiven Bestandteil umfaßt.

8. Pharmazeutische Zusammensetzung nach Anspruch 7, worin der Ester Fumagillin ist.

9. Pharmazeutische Zusammensetzung nach Anspruch 7 oder 8, welche 1 bis 200 mg aktiven Bestandteil enthält.

10. Pharmazeutische Zusammensetzung nach Anspruch 9, welche 7,5 bis 30 mg aktiven Bestandteil enthält.
